# EUROPEAN PATENT APPLICATION

(11) **EP 4 443 066 A1**
(43) Date of publication of application: **09.10.2024**
(21) Application number: 23166385.7
(22) Date of filing: 03.04.2023
(51) Int. Cl.: F24F 8/20, A61G 13/10, A61L 9/00

(54) **SYSTEM FOR AIR DECONTAMINATION**

(71) Applicant: NanoCleanAir GmbH, 5443 Niederrohrdorf (CH)
(72) Inventor: Mayer, Andreas, 5443 Niederrohrdorf (CH); Czerwinski, Jan, 2562 Port (CH); Burtscher, Heinz, 5420 Ehrendingen (CH); Mayer, Jörg, 5702 Niederlenz (CH); Lutz, Thomas, 8132 Egg (CH); Jent, Philipp, 3010 Bern (CH)
(74) Representative: Troesch Scheidegger Werner AG

(57) **Abstract**

The present invention pertains to a system for air decontamination of a three-dimensional space as well as to a corresponding kit of part, a use of the system and a method for decontamination of a three-dimensional space.

## Description

The present invention pertains to a system for air decontamination of a three-dimensional space as well as to a corresponding kit of parts, a use of the system and a method for decontamination of a three-dimensional space.

Ultrafine particles, also called nanoparticles, include pollutants and pathogens such as viruses which may remain in a floating state in ambient air with a remarkably slow rate of decent (about 10 cm/h for viruses) while remaining infective over significant periods (about 3 to 5 hours to a few days for viruses). The health risk for humans and animals being exposed to, e.g., viruses is especially significant in indoor facilities. Furthermore, the exposure to airborne pathogens in facilities for people with impaired health, such as in hospitals or nursing homes, is often associated with the spread of infections and increased rates of diseases and mortality. At the same time, it is impractical and cost-intensive to isolate the vulnerable (e.g. immunocompromised) or the infected patients spatially in hospitals or nursing homes due to space restrictions. It is commonly accepted that the aerating of rooms by opening windows or by generally increasing ventilation capacity (e.g. of existing air conditioning systems) is not effective in preventing the spread of infections in rooms by airborne pathogens such as viruses (W. G. Lindsley et al., MMWR Morb. Mortal. Wkly. Rep. 2021; 70(27): 972-976; L. H. Saw et al., Aerosol and Air Quality Research 2022; 22(3): 210213).

It is the objective of the present invention to provide an improved system for decontaminating and optionally isolating a space from airborne ultrafine particles.

The above problem is solved by the system according to claim 1.

In a first aspect, the present invention is directed to a system for air decontamination of a three-dimensional (3D) space having a top and a bottom being offset in direction of gravity with respect to the top, the system comprising
(I) at least one canopy comprising a canopy top positioned essentially at the top of the 3D space and bordered by a circumferential bordering element extending from the canopy top towards the 3D space, wherein the canopy top and the bordering element define a partial volume of the 3D space,
(II) at least one air inlet member positioned in the partial volume, and configured to draw in air from the partial volume of the 3D space and optionally from the 3D space, and
(III) an air displacement device configured to move air from the at least one air inlet member through a filter device and through at least one air outlet into the 3D space, optionally into the 3D space essentially at the bottom of the 3D space, wherein the filter device is configured to filter ultrafine particles from the air drawn in through the at least one air inlet member and optionally filter ultrafine particles from fresh air,
wherein the system is configured to draw air in a substantially laminar flow essentially in direction of the bottom of the 3D space to the top, optionally essentially from the bottom to at least the partial volume.

The system of the present invention was found to be surprisingly effective in decontaminating and confining a 3D space by exchanging the air of the 3D space by filtered air and by creating a laminar flow within the 3D space in direction of the top of the 3D space.

For example, the system of the present invention can be configured such that a disturbance of the substantially laminar flow, e.g. by a person walking into the room or by other disturbing air flows, can be overcome by restoring the laminar flow within a short period of time, e.g. within at most 10 mins or less.

The 3D space is the space that is decontaminated and optionally isolated by the system of the present invention. The dimensions of the 3D space are defined by
- the height of the present system (y-axis from the bottom to the top, wherein the bottom of the 3D space is offset in direction of gravity with respect to the top) from the bottom-most part of the system, e.g. a floor, and/or the air outlet of the system to the canopy top, and
- the area defined by the canopy (x- and z-axis).

The area of the canopy corresponds to the area of the canopy top if the circumferential bordering element is angled at 90 degrees relative to the canopy top and if the canopy top is positioned at 90 degrees relative to gravity. If the angle of the circumferential element is smaller or greater than 90 degrees, and/or if the canopy top is angled other than 90 degrees relative to gravity, the area of the 3D space equals the projection of the canopy (including any circumferential bordering elements angled at more or less than 90 degrees relative to the canopy top) in direction of gravity on a plane that is perpendicular to gravity. Different angles for the circumferential bordering element and the projection are exemplified in the Figures below.

Exemplary volumes for the 3D space, as used herein, include, e.g., 1 to 2 m³, 3 to 6 m³, 8 to 30 m³, or 10 to 20 m³. n-multiples of these values apply if n-systems according to the present invention are combined side by side essentially without any distance between the systems.

For example, if the system is configured to receive a bed in the 3D space, the volume of the 3D space can, e.g., be about 2x2x2 m to 3x3x3 m, or (area times height) 2.5x2x2 to 3x2.5x2.5

The partial 3D space, as used herein, is the part of the (same) 3D space that is located in the volume of the canopy, i.e. the volume defined by the canopy top and the circumferential bordering element. Exemplary illustrations of the 3D space and the partial 3D space are given in the below Figures.

For example, the partial volume of the 3D space can make up 5 to 30 %, optionally 10 to 20 % of the 3D space, optionally 0.1 to 7 m³ or 0.5 to 2 m³.

The percentage of the partial volume also depends on what type of object or person the system is configured to receive in the 3D space. If the system is configured to receive a person on a chair, the partial volume can be in the range of 0.1 to 1 m³ or 0.5 to 1 m³, and for a person on a double bed, the partial volume can be in the range of 0.5 to 4 m³.

The substantially vertical laminar flow, i.e. a relatively slow and essentially turbulence-free air flow, effectively carries particles up and away from a person in the 3D space. This laminar flow essentially leads to a stratification of the air in space of which at least the top layer is prevented from lateral or vertical movement, and is removed through the at least one air inlet member. The term "laminar flow" as used herein is understood as commonly known in the art, i.e. referring to gas particles following smooth paths in layers, with each layer moving smoothly past the adjacent layers with little or no mixing. The laminar upwards flow is supported by the natural thermal effect, e.g. the convective flow of air, e.g. around a person who naturally radiates body heat (or around other warm objects). The laminar flow not only removes contaminated air up and away, it also forms a screen to the space outside the 3D space and at least significantly reduces contamination of the outside space from within the 3D space. Also, a contamination of the 3D space from the outside space is significantly reduced.

The substantially vertical laminar flow is at least assisted by the natural propensity of air to flow upwards. The natural body heat of persons (about 100 watt per person), and/or the addition of heat to objects or the heating of the air admitted to the 3D space (e.g. 3 to 30°C, 5 to 20°C, optionally 3 to 10°C or about 10°C above room temperature) can further and thermally assist the substantially vertical laminar flow towards the partial volume of the 3D space and towards the air inlet member(s) therein.

Without wishing to be bound by theory, the system of the present invention can create a pressure gradient along the axis of gravity within the 3D space, wherein the relative lower pressure is located within the partial 3D volume, in particular around the at least one air inlet, and a relative higher pressure is located within the 3D system, optionally increasing in direction of gravity to the bottom of the 3D system or the air outlet(s) of the system. This pressure gradient can assist the laminar flow of air, e.g. together with thermal convection, to overcome the force of gravity and move in the opposite direction of gravity into the partial 3D space and/or the at least one air inlet of the system.

It is noted that the substantially vertical laminar flow is not primarily the result of or caused by the air exiting the air outlet(s). In other words, the air outlets are not necessarily arranged such that they actively "blow" air upwards to cause a flow towards the air inlet member(s). In other words, the air outlets do not need to be positioned opposite the air inlets (in vertical direction along gravity) but can be positioned anywhere as long as their position allows for a substantially vertical laminar flow to occur towards the partial volume of the 3D space and the air inlet member(s), e.g. around a person within the 3D space. Optionally the air outlets are positioned in the lower part of the 3D space or below a bed positioned within the 3D space.

The canopy for use in the present system can be of any shape, e.g. round, oval, square, convex, concave etc. For example, the canopy (i.e. the canopy top and the circumferential bordering element) can be transparent, e.g. made from glass or a transparent plastic, e.g. plexiglass. The exemplary transparent nature of the canopy has the advantage that light emitted, e.g. by lamps, above the canopy can reach the 3D space and no or not much additional lighting is required.

In an alternative example, the canopy can be made of a fabric that can either be air-tight or not air-tight, depending on how the pressure within the partial volume of the 3D space is adjusted, a slight loss or intake of air via the canopy can be tolerated. If the canopy is not transparent, lighting means can be attached to the canopy or within the canopy to other components of the system.

For example, the canopy top can have a length of about 1 to 4 m, optionally 2 to 3 m, and/or a width of about 1 to 4 m, optionally 2 to 3 m. n-multiples of these values apply if n-systems according to the present invention are combined side by side essentially without any distance between the systems.

For example, the circumferential bordering element can have a length (the distance from the canopy top away from the canopy top) of about 5 to 50 cm, optionally 10 to 30 cm.

For example, the canopy top and/or the circumferential bordering element can have a thickness of about 0.5 to 10 mm, optionally about 1.0 to 3 mm.

For example, the canopy for use in the present system can be essentially air-tight (made from air-tight materials) in opposite direction of gravity and in directions perpendicular to gravity so that air does essentially not escape the partial volume of the 3D space in the aforementioned directions. Alternatively, the canopy can be made from a material (e.g. a membrane) which allows for air to enter the partial volume but not to exit the partial volume. Alternatively, the canopy can be air-permeable to the inside and outside of the partial volume. The air-permeability can be adjusted to the extent that the laminar flow of air from the 3D space into the partial volume of the 3D space and into the air inlet members is not impaired by the air-permeable properties of the canopy. For example, a certain air-permeability of the canopy (e.g. as a directional membrane forming at least a part of the canopy, e.g. the canopy top) can allow for a defined and limited amount of "fresh" air from the outside of the 3D space to enter the partial volume and the system. Due to the air inlet members, the system may feature a lower pressure within the canopy/in the partial volume of the 3D space compared to the space outside the system and the 3D space, which supports the substantially laminar flow and allows for air to additionally enter the partial volume through the canopy.

The substantially laminar flow within the 3D space moves into the partial volume of the 3D space defined by the canopy and the air that moved into the partial 3D space is at least partially and at least temporarily "collected" therein before it is removed by the at least one air inlet member. The "collection" of the air arriving in the partial volume of the 3D space via the substantially laminar flow can also, e.g., be assisted by the relative lower pressure in the partial volume of the 3D space compared to a relative higher pressure outside the partial volume of the 3D space (but still within the 3D space) caused by the at least one air inlet member drawing in air.

The at least one air inlet member for use in the present system can be any member that is suitable for taking up air, including rectangular hollow beams, (round) tubes and mixtures thereof. The air inlet members may include one or multiple air inlets for drawing in air into the air inlet members. The air inlet member may also, in addition, be a structural member that is capable of supporting itself and optionally the canopy.

The at least one air inlet member is positioned in the partial volume defined by the canopy. It may be positioned at any location within the partial volume, and it is not necessarily located at the top of the partial volume.

The at least one air inlet member can draw in air from the partial volume of the 3D space and optionally from the (bordering) 3D space. It can also draw in air that is "collected" and was temporarily stored within the partial 3D space by drawing in air between the at least one air inlet member and the canopy top. The air inlet member may be positioned such and have suitably positioned air inlet openings that it can draw in air that has accumulated between the air inlet member and the canopy top.

The at least one air inlet member is in fluid communication with the filter device, the at least one air outlet, and with the air displacement device. Fluid communication can be achieved by connecting the aforementioned components by means dimensionally stable or by flexible ducting of any shape and diameter/area.

The air displacement device can be any device suitable for moving air, e.g. a radial blower or a fan. The air displacement device can, e.g., also be a device that moves air based on inducing a temperature difference, e.g. to cool the air that rose into the partial 3D space in order for it to sink and/or to heat air, e.g. at the air outlet, in order for it to raise or at least to assist the substantially laminar flow. A device inducing a temperature difference can be used in addition to another air displacement device, e.g. a radial blower or fan.

For example, a radial blower can be used as an air displacement device, e.g. with steep pressure characteristics, e.g. a radial blower that provides a pressure level of about 2 mbar or up to about 25 mbar. For example, the air displacement device can be positioned before the filter and can push air onto/through the filter while drawing air from the air inlet member(s) and optionally from a fresh air source. Of course, multiple, e.g. two, air displacement devices can be present in the system described herein.

Fresh air can be administered to the system and the 3D space described herein, optionally fresh air that is filtered by the filter device described herein before being administered to the 3D space. Also, moisture can be added to or removed, e.g. condensed, from the air drawn through the system. The amount of fresh air or moisture administered into the system of the present invention, e.g. by the air displacement device, can be regulated, e.g. in order to adjust moisture, CO₂ and/or O₂ levels within the 3D space. The fresh air as well as the recirculated and filtered air can also be cooled or heated by suitable cooling or heating means. Also, a therapeutic gas, aromas or aerosols can be added to the air that is administered to the 3D space, e.g. for asthma patients.

At least part of the air drawn in through the at least one air inlet member can, e.g., also be exhausted to the outside of the 3D space, optionally after passing the filter device. The amount of exhausted air can be controlled, e.g., based on the air quality in the 3D space, e.g., according to CO₂ levels. It is noted that the CO₂ and O₂ levels can also, e.g., be levelled by a certain level of diffusion between the outside space and the 3D space.

The system can comprise suitable means for measuring air quality, e.g., measuring moisture, CO₂ and/or O₂ levels.

For example, the at least one air outlet for use in the present system can be positioned essentially below the height of a person standing, sitting on a chair or lying on a bed in the 3D space, or on the bottom of the 3D space. The substantially laminar flow is then predominantly supported from the location of the air outlet to the partial 3D space. The at least one air outlet is positioned such that it at least assists the air to flow in a laminar flow substantially to the partial volume of the 3D space, in other words, the air outlet(s) is(are) positioned in the system such that its/their position together with the speed of the air exiting the air outlet allow for and optionally support the substantially vertical laminar flow, for example from below a person positioned in the 3D space (be it standing, sitting on a chair or lying on a bed) or from the bottom of the 3D space into the air inlet member(s).

The at least one air outlet can be porous (comprise pores), e.g., can comprise a mesh or a porous membrane through which the filtered and optionally fresh air is administered to the 3D space. The term "porous membrane", as used herein, refers to any mono- or multi-layered structure that is permeable to air and optionally reduces the velocity of the air passing through the membrane upon administration to the 3D space. The pores of the membrane can be of any size, shape and number as long as they allow air to pass such that the substantially laminar flow in the system is supported, e.g. by admitting air in an essentially turbulence-free and/or laminar flow. The membrane may comprise one or more layers of one or different materials, e.g. to achieve sufficient structural stability to withstand the pressure difference between the inside (air outlet) and the outside (3D space), and/or to reduce the air velocity.

For example, the porous membrane can be configured to reduce a velocity of the filtered and optionally fresh air upon administration to the 3D space by a factor of about 50 to 200, optionally by a factor of about 50 to 100. Suitable materials for the membrane are known in the art. Membranes with sufficient stability include, e.g. sieve or screen cloths/fabrics or mesh laminates which are typically used in the context of sieves or in the filter industry (e.g. as provided by PACO Paul GmbH & Co. KG Metallgewebe und Filterfabriken, Steinau, Germany). Depending on the application, the membrane can comprise a structural support layer such as, e.g., a perforated (metal) plate.

For example, the air outlet can be covered by a perforated plate with, e.g., a hole density of about 8 to 10%, optionally about 8.8%.

In the present system, at least part of the filtered air is exhausted through the at least one air outlet into the 3D space. "At least part of the filtered air" includes that air can be alternately exhausted to the outside of the 3D space and to the inside of the 3D space. It is advantageous to administer enough filtered air into the 3D system in order to avoid a negative pressure within the 3D space (excluding the partial volume of the 3D space), e.g. at a lower part of the 3D space, so as to prevent air entering from the outside into the 3D space.

The term "air decontamination" as used herein refers to filtering air from at least ultrafine particles. Ultrafine particles are particles of a mean diameter size of 10 to 500 nm, in particular 30 to 200 nm. Optionally the air comprises less than about 5000 ultrafine particles per cm³ of air, optionally less than about 2000 or 1000 ultrafine particles per cm³ of air, after being filtered (after passing the filter device). Additionally, larger particles such as dust, pollen, coarse aerosols and bacteria, e.g. having mean diameters of more than 1 µm or 500 nm can be, optionally, filtered. The system of the present invention can be configured to continuously recycle the air within the 3D space which, e.g., with every cycle of recirculation, leads to a further reduction in contamination. A limited diffusion of air from the space outside the 3D space into the 3D space can occur which may lead to the addition of "fresh air", however, this air from outside is also decontaminated by the system as soon as it enters the filter device via the at least one air inlet member.

The filter device for use in the present system can be any filter capable of filtering ultrafine particles, e.g., as described in Handbuch Verbrennungsmotor by Basshuysen /Springer Vieweg 2017 Chapter 21.6.3. The filter device can, e.g., be a cell-type, e.g. a honeycomb filter, e.g. as described in the SAE-Paper SAE 2009-01-1087. The filter device may be a wall flow filter featuring alternatingly closed and open cells which forces the air to pass the cell walls, resulting in a high filtration surface and low filtration speeds. An exemplary filter is described in the SAE paper of CORNING 2021-01-0584. The filter device may be coated with an antimicrobial, antifungal and/or antiviral coating, e.g. a silver- or copper-comprising coating. Additionally or alternatively, the filter device may be coated with a hydrophobic material on the air entry side and/or with a hydrophilic material on the air exit side. Of course, multiple filter devices may be present and, for example, one filter device can be present for one air displacement device, wherein the filter is optionally positioned at the air outlet side of the air displacement device. Typically, the filter device can be made from ceramic materials such as, e.g., Cordierit. The filter device may further comprise heating means for heating and sterilizing the filter.

In general, for all embodiments and aspects described herein, more than one of the components of the system described herein can be present in the system and the term "a" does not limit the number to one.

In an embodiment of the system according to the present invention, which may be combined with any of the embodiments of the system still to be addressed unless in contradiction, the at least one air inlet member is positioned essentially at the canopy top and is optionally at least partially attached to the canopy top.

Alternatively, the canopy top (and/or the canopy) can be supported independent of the air inlet member by other structural means so as not to have physical contact with air inlet member or any other part of the system described herein. Alternatively, the canopy or canopy top can be attached to a ceiling above the canopy, e.g. a ceiling of a room in which the system is positioned.

In an embodiment of the system according to the present invention, which may be combined with any of the embodiments of the system still to be addressed unless in contradiction, the at least one air inlet member is formed by tubes, optionally positioned essentially in parallel to the canopy top, optionally by tubes comprising openings (e.g. pores) distributed over 120 to 360 degrees around the tubes, for drawing in air.

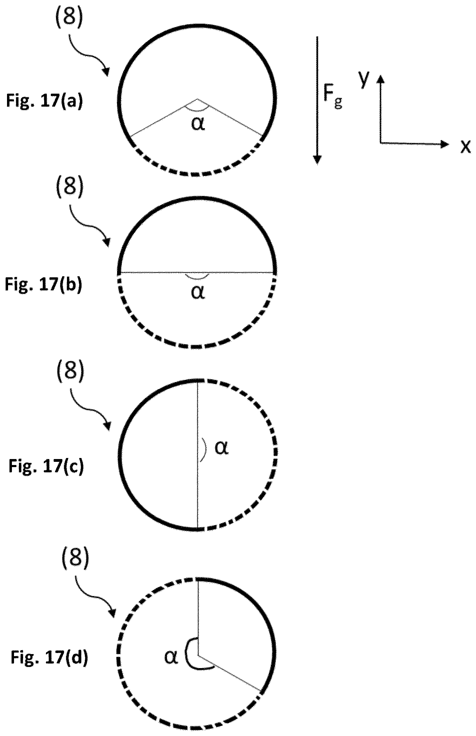

The openings (e.g. pores) in the tubular air inlet member can be distributed around the circumference of the inlet member. If the openings are distributed over about 120 to 179 degrees (i.e. at about 120 to 179 degrees of the 360 degree circumference, see, e.g., Figure 17), it is preferred that the openings are positioned such that they face the 3D space and not the canopy top. The distribution of the openings is optionally such that air can be drawn in from a direction facing the canopy top, as well as from a direction facing the 3D space (direction bottom of the 3D space, see, e.g., Figure 17). The tubular air inlet member may comprise sections with and sections without openings. The skilled person will appreciate that the distribution of air intake can be adjusted by the location of the openings, optionally together with the location of the (tubular) air inlet member within the partial 3D space, according to the desired distribution of air intake. For example, the distribution of openings around 180 to 270 degrees around the circumference of the tubular air inlet member has the further advantage of taking in air from different directions (e.g. in direction canopy top, circumferential bordering element and in direction bottom of the 3D space) while still warranting structural rigidity to the tubular air inlet member.

In an embodiment of the system according to the present invention, which may be combined with any of the embodiments of the system still to be addressed unless in contradiction, the system is configured to draw in about 100 to 200 m³ of air per hour.

For example, if the system is configured to receive a person in the 3D space, the system can be further configured to draw in about the 200- to 360-fold amount of the person's respiratory volume per hour (for more than one person, the numbers are multiplied accordingly). The person's respiratory volume is optionally about 0.5 m³ per hour.

In an embodiment of the system according to the present invention, which may be combined with any of the embodiments of the system still to be addressed unless in contradiction,
- the system is configured so that the substantially vertical laminar flow has an average speed of about 0.002 to 0.04 m/s, optionally 0.005 to 0.02 m/s,
- the total area of all openings in the air inlet member for air intake, optionally the sum of all opening cross-sections in the air inlet member, is about 0.009 to 0.036 m², optionally about 0.0015 to 0.025 m², for a volumetric flow within the system of about 45 to 180 m³/h, optionally for about 80 to 120 m³/h, and/or
- the total area of all openings in the air outlet for air outlet, optionally the sum of all opening cross-sections in the air outlet, is about 0.0135 to 0.054 m², optionally about 0.015 to 0.03 m², for a volumetric flow within the system of about 45 to 180 m³/h, optionally for about 80 to 120 m³/h.

The total area of all openings in the air inlet member for air intake refers to all openings in the inlet member which draw in air from the partial volume of the 3D space and/or the 3D space. The openings may have any shape, however, if they are round openings, the total area refers to the sum of all opening cross-sections. Optionally, the openings in the at least one air inlet member for drawing in air have an area of about 2 to 4 mm², optionally a diameter of about 1 to 4 mm or about 1.5 to 2.5 mm for round openings.

The total area of all openings in the air outlet for air outlet refers to all openings in the air outlet which exhaust air into the 3D space and optionally into the outside of the 3D space.

The openings may have any shape, however, if they are round openings, the total area refers to the sum of all opening cross-sections. Optionally, the openings in the outlet for exhausting air have an area of about 2 to 4 mm², optionally a diameter of about 1 to 4 mm or about 1.5 to 2.5 mm for round openings.

The average speed of the substantially vertical laminar flow is the mean or average speed the air flow has as measured in the 3D space.

The skilled person can adjust the total area of all openings of the air inlet member and air outlet and can adapt the volumetric flow which the system achieves by structural means and by adjusting the air flow, e.g. by adjusting the flow generated by the air displacement device, e.g. in accordance with the above values.

For example,
- the system can be configured to have an average air speed of about 0.005 to 0.2 m/s, optionally about 0.02 to 0.1 m/s at the at least one air inlet member,
- the system can be configured to have an average air speed of about 0.01 to 0.4 m/s, optionally about 0.05 to 0.2 m/s at the at least one air outlet.

The average air speed at the at least one air inlet member is measured as average value at the surface comprising openings for air intake (e.g. the porous surface) of the at least one air inlet member. The average air speed at the at least one air outlet refers to the speed of the air when it leaves the air outlet. The air speed within the at least one air outlet may be higher than the speed of the air leaving the air outlet. For example, means can be installed for reducing the air speed at the interface between the air outlet and the outside, e.g. 3D space, e.g. as described above, e.g. in the context of membranes.

In an embodiment of the system according to the present invention, which may be combined with any of the embodiments of the system still to be addressed unless in contradiction, the at least one air inlet member is formed by tubes comprising openings, optionally distributed over about 120 to 360 degrees around at least parts of the tubes, wherein
- the porosity of the tubes formed by the openings is about 0.5 to 2 %,
- the openings have an area of about 2 to 4 mm², optionally a diameter of about 1 to 4 mm or about 1.5 to 2.5 mm for round openings,
- the total volume of the at least one air inlet member formed by tubes is about 2.5 to 5 % of the volume of the 3D space, and/or
- the cross-sectional area of the at least one air inlet member tube is about 0.5 to 2 times larger than the total area of the openings.

The openings in the at least one air inlet member refer to the openings in the inlet member which draw in air from the partial volume of the 3D space and/or the 3D space.

In an embodiment of the system according to the present invention, which may be combined with any of the embodiments of the system still to be addressed unless in contradiction, the at least one air inlet member forms a support structure for the at least one canopy and optionally wherein the canopy top and/or the circumferential bordering element is/are transparent. Alternatively, the canopy top and/or the circumferential bordering element is/are not transparent and/or the system optionally further comprises lighting means to illuminate the 3D space.

In an example, the at least one air inlet member supports itself and/or further supports (e.g. carries through attachments) the canopy, wherein the canopy is not, e.g., attached to any other structure than the at least one air inlet member, e.g. is not attached to a ceiling of a room in which the system is positioned, and/or is not supported by a support structure such as a pillar positioned on the bottom of the 3D space.

In an embodiment of the system according to the present invention, which may be combined with any of the embodiments of the system still to be addressed unless in contradiction, the canopy top is essentially flat, and/or optionally the circumferential bordering element extends at an angle of about 45 to 135 degrees, optionally about 90 degrees, relative to the canopy top.

For example, the circumferential bordering element can have a height of more than about 5 or 10 cm, optionally of less than 40, 30 or 20 cm (i.e. 5 or 10 to 40, 30 or 20 cm, measured as the smallest distance from the point of attachment to the canopy top to the rim of the circumferential bordering element). The position of the angles and distances indicated above are illustrated in the Figures.

In an embodiment of the system according to the present invention, which may be combined with any of the embodiments of the system still to be addressed unless in contradiction, the at least one canopy, optionally the at least one air inlet member optionally attached to the canopy top and/or the circumferential bordering element, is connected to at least one essentially vertical support tube for positioning and supporting the at least one canopy above the bottom of the 3D space.

The at least one essentially vertical support can have any shape suitable for supporting the at least one air inlet member and/or canopy, e.g. round or rectangular, e.g. a tube. For example, the at least one vertical support can be hollow to provide for a passage for the air taken in through the at least one air inlet member. For example, the at least one vertical support can support the at least one air inlet member which in turn supports the canopy.

The at least one essentially vertical support may further comprise noise reduction means within the support to reduce the noise of the air passing through the vertical support, e.g. from the air inlet member, optionally via the air displacement device, to the filter device and air outlet. The noise reduction means can be any means for reducing the noise of an air stream, e.g. an absorption system, a reflection system or a resonance system.

For example, the canopy and/or at least one air inlet member may be connected to the vertical support by means of a hinge which allows for the canopy and/or the at least one air inlet member to be folded in a parallel position to the vertical support. The folding may aid in transporting the system, e.g. within a building through doors. Additionally or alternatively, the vertical support(s) can be telescopic, detachable or foldable, e.g. in order to reduce the height of the system, e.g. for transportation.

In an embodiment of the system according to the present invention, which may be combined with any of the embodiments of the system still to be addressed unless in contradiction, the vertical support supports and fluidly connects the at least one air inlet member with a housing comprising the filter device and the air outlet and optionally the air displacement device, wherein the housing is optionally positioned on the bottom of the 3D space.

The housing can, e.g., be constructed such as to form a stand for the system of the present invention, and it can, e.g., comprise further stabilizing means such as one or more arms for stabilizing the system on a ground at the bottom of the 3D space. The housing may further comprise means for transporting or moving the housing, e.g. wheels or lockable wheels.

The means for noise reduction in the housing can be the means defined above in the context of the vertical support. The means for noise reduction may act as resonance or absorption elements, wherein the absorption layers are adapted in their geometrical depth (optionally about 30 to 100 mm) and/or density to the frequency response in order to, e.g., reduce harmonic small band noise (e.g. harmonic or fan blade frequency) and optionally create a sound spectrum that is more pleasant for the human ear. For example, the means for noise reduction can be located after the air displacement means and/or after the filter device in direction air flow. For example, the means for noise reduction can be located after the filter device and the filter device itself can be a means for noise reduction. Additionally, and for example, means for noise reduction can be located upstream before the air displacement means and the filter, e.g., within the vertical support.

In an embodiment of the system according to the present invention, which may be combined with any of the embodiments of the system still to be addressed unless in contradiction, the vertical support and/or the housing comprise(s) means for noise reduction, optionally upstream and downstream of the air displacement device, optionally wherein the means for noise reduction in the housing are configured to deflect the air exiting the filter device and/or the air displacement device by about 180 degrees through the means for noise reduction to the air outlet.

The air may also be deflected at less or more than 180 degrees in other directions, e.g. in order to increase the distance the air travels through the noise reduction means. For example, the noise reduction means in the vertical support and/or the housing may be configured to achieve a noise level emitted by the system of less than about 40 dB(A) as measured in the 3D space, optionally in the center of the 3D space.

In an embodiment of the system according to the present invention, which may be combined with any of the embodiments of the system still to be addressed unless in contradiction, the system is configured to accommodate a bed in the 3D space, optionally wherein the housing is configured to be positioned behind and below the head section of the bed.

In an embodiment, the air outlet and optionally the housing are positioned such that, if a bed is installed in the 3D space, e.g. a hospital or care home bed, the air exits the air outlet essentially centrally under the bed, e.g. in order to provide a symmetrical air distribution around the bed.

For example, two individual air inlet members may be positioned so as to introduce a symmetry axis in the 3D space, e.g. to "divide" the 3D space into to half spaces above each of which one air inlet member resides. The air inlet members may also be arranged such as to increase the number of air inlet members along a central axis, or a central area, of the 3D space, e.g. to double the number of air inlet members along a central axis or central area of the 3D space compared to the periphery of the 3D space. The central axis or central area can correspond to the position of the contamination source in the 3D space. If the contamination source is located at other positions within the 3D space, the number of air inlet members can be increase over those positions. For example, the difference in air intake can be achieved by the distribution of openings in an air inlet member formed by a tube and/or by increasing the number of air inlet members or tubes in the area where a higher air intake should be achieved.

For example, the air inlet members can be arranged and positioned such that a difference in suction power between different positions in the partial 3D space is not greater than five-, optionally two-fold. In other words, the air inlet members are optionally positioned such that the difference in suction power at different positions within the partial 3D space does not impede the substantially vertical laminar flow, e.g. by causing relevant transverse air flows or turbulence.

In an embodiment of the system according to the present invention, which may be combined with any of the embodiments of the system still to be addressed unless in contradiction, the system comprises
- two air inlet members each formed by a tube loop and optionally comprising openings distributed over about 120 to 360 degrees around at least part of the tube,
- the canopy supported by the two air inlet members,
- two vertical supports each fluidly connected to and supporting one of the two air inlet members and both further fluidly connected to
- the housing comprising the filter device and the air outlet, and optionally the air displacement device.

In the above embodiment, the system can be redundant in that each of the two air inlet members together with the vertical support tubes can each be connected to a filter device and optionally to an air displacement device. In case one of the components fail, e.g. one filter device or one air displacement device, the system remains functional through the other filter device or air displacement device and the laminar flow is at least partially upheld.

In an embodiment of the system according to the present invention, which may be combined with any of the embodiments of the system still to be addressed unless in contradiction,
- the two vertical supports each define an adjacent corner of the 3D space and together with the housing and the canopy essentially define the height of the 3D space; and/or
- the canopy top, the circumferential bordering element, and/or the at least one air inlet member is/are detachably, pivotably or foldably connected to each other and/or to the at least one essentially vertical support.

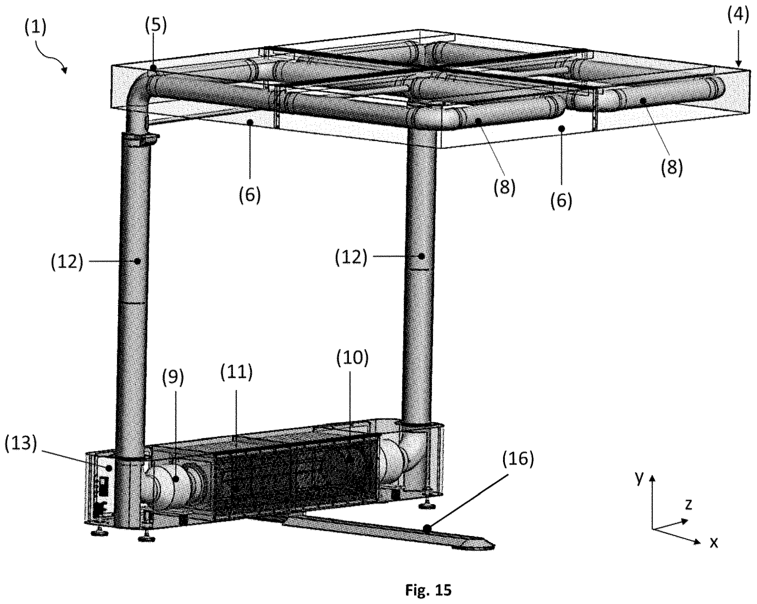

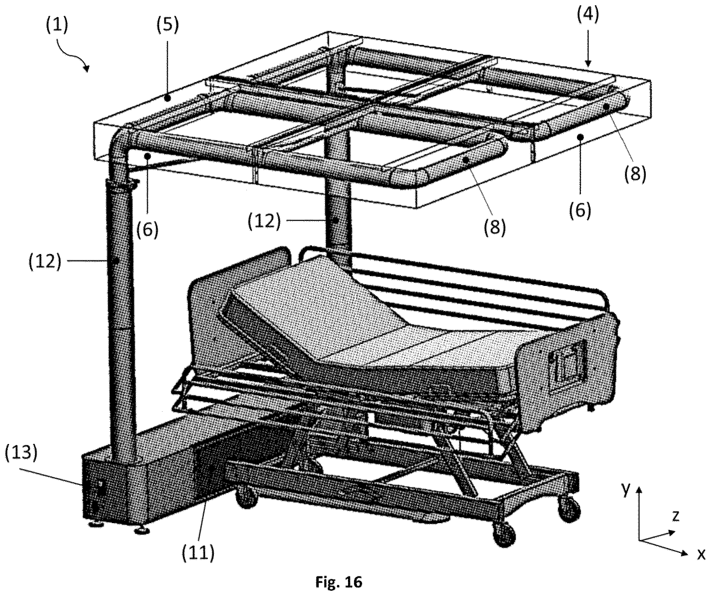

For example, the system may comprise one or more additional support member(s) configured to prevent the system from tilting, e.g. in direction of an overhang of the canopy, e.g. as shows in Figures 15 and 16. These optional additional support members may, e.g. be attached to the housing or vertical support(s) and may also further prevent tilting of the system if, e.g., a force (e.g. up to 400 N) in direction of gravity is applied to the periphery of the canopy, e.g. by a person pulling.

As detailed above, the system of the present invention is essentially dimensioned such that at least one person (standing, sitting or lying, including a corresponding stool or bed) can be positioned within the 3D space. Optionally 2, 3, or 4 people (standing, sitting or lying, including a corresponding number of stools or beds) can be positioned within the 3D space. Also, in an embodiment, the system is dimensioned such that the system is transportable within a building (a hospital or care home), optionally after folding down or removing the canopy and optionally the air inlet member(s). The vertical supports can be telescopic in order to reduce the height of the system, e.g. for transportation and the system may be supported by (e.g. lockable) wheels, e.g. on the housing.

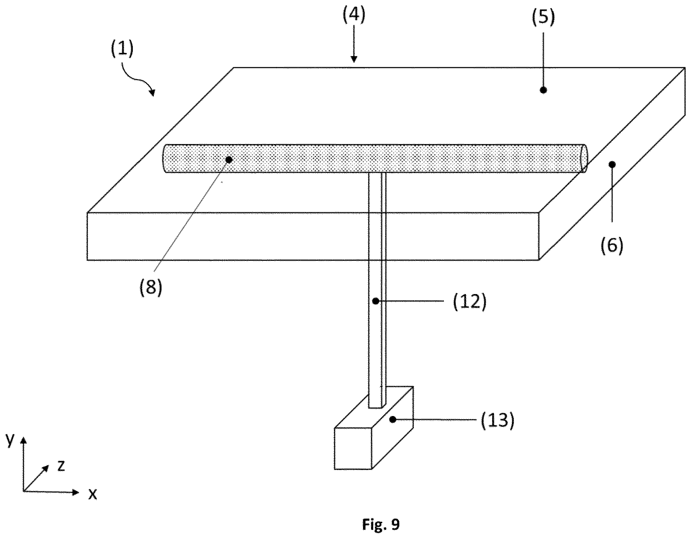

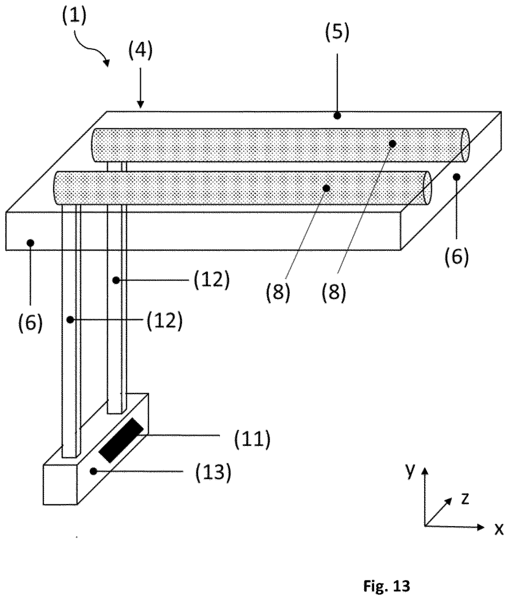

Non-limiting and exemplary dimensions of the system in the assembled state include:
Height: from about 1 m to about 3 m, optionally between 1.9 and 2.5 or 2.2 m;
Width (z-axis as defined, e.g., in Figures 9 or 13): from about 1.5 m to about 5 m, optionally about 1.8 to 2.5 m;
Length (x-axis as defined, e.g., in Figures 9 or 13): from about 1.7 to about 5 m, optionally about 1.8 to about 2.5 m.

The components of the present system, in particular the air inlet members, the vertical support and any other components (e.g. also the canopy) which are in contact with the contaminated and decontaminated air moved through the system can be coated with an easy to clean and cleaning composition-resistant material (e.g. alcohol-resistant), e.g. polymer powder coatings, optionally based on polypropylene, polyamide, or acrylonitrile butadiene styrene, optionally extruded or pressed, uncoated and optionally polished or anodized metal surfaces. The structural components of the present system, in particular the air inlet members, the vertical support and the housing can be made of, e.g. steel or alloys with sufficient strength/stability, or from a steel /alloy framework providing stability combined with polymer or metal sheet covers for outer surfaces and air flow guidance.

Further objectives are achieved by a kit of parts according to claim 15, by a use and by a method as described below.

In another aspect which may be combined with any of the embodiments or aspects preaddressed or still to be addressed unless in contradiction, the present invention is directed to a kit of parts comprising the components of the system as described herein, and optionally instructions for their assembly and/or the use of the system.

In another aspect which may be combined with any of the embodiments or aspects preaddressed or still to be addressed unless in contradiction, the present invention is directed to a use of the system for decontamination of a 3D space, optionally a 3D space in a hospital or care home.

In another aspect which may be combined with any of the embodiments or aspects preaddressed or still to be addressed unless in contradiction, the present invention is directed to a method for decontamination of a 3D space within a hospital or care home comprising the steps of (i) installing a system as described herein and (ii) activating the air displacement device of the system.

It is emphasized that all the above embodiments and aspects may be combined with each other unless in contradiction.

### Brief description of the Figures:

- Figures 1a,b: Show a canopy and the partial 3D space.
- Figures 2a,b: Show a 3D space, partial 3D space and laminar air flow.
- Figures 3a-g: Show a canopy and air inlet members.
- Figures 4a-d: Show a canopy and air inlet members.
- Figures 5a,b: Show a system including one vertical support and one canopy.
- Figure 6: Shows a system including one vertical support and one canopy and the corresponding 3D space, and partial 3D space.
- Figure 7: Shows a system including one vertical support and one canopy and the corresponding 3D space, and partial 3D space.
- Figure 8: Shows the area of the canopy (top) and the 3D space.
- Figure 9: Shows a system including one vertical support and one canopy.
- Figure 10: Shows a system including one vertical support, one canopy and two beds.
- Figure 11: Shows a system including two vertical supports, one canopy and an arrangement of air inlet members.
- Figure 12: Shows a system including two vertical supports, one canopy, an arrangement of air inlet members and beds.
- Figure 13: Shows a system including two vertical supports, one canopy and an arrangement of air inlet members.
- Figure 14a,b: Show a system including two vertical supports, one canopy and an arrangement of air inlet members.
- Figure 15: Shows a system with a transparent housing.
- Figure 16: Shows a system in relation to a bed.
- Figure 17: Shows an exemplary opening distribution on an air intake member.
- Figures 18a-c: Show an exemplary arrangement of noise reduction means and vertical support, and an exemplary air inlet member.
- Figure 19: Shows an exemplary arrangement of noise reduction means and air flow.
- Figure 20: Shows the reduction of particles in the system.

In the following, the invention will be illustrated by reference to Figures which are not intended to limit the scope of the invention as described in the appended claims. In all Figures, the coordinate system is indicated and the opposite direction of the y-axis (-y) corresponds to gravity.

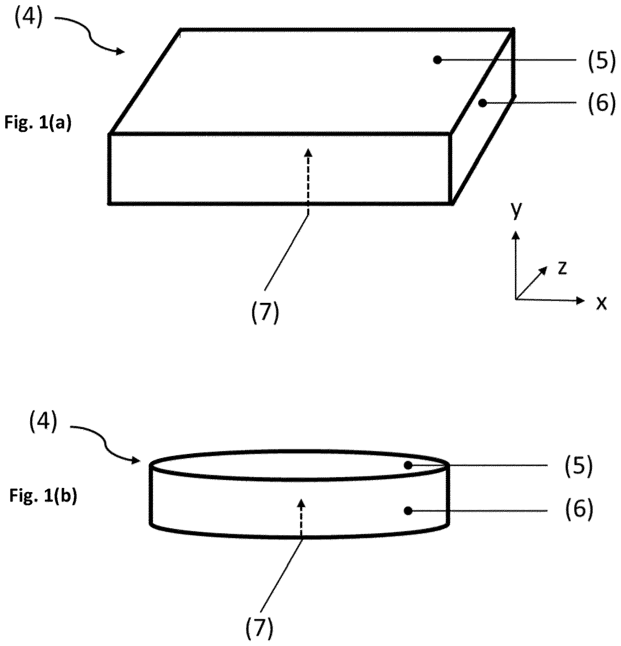

Figure 1(a) shows a canopy (4) for use in the present system (1) with the canopy top (5) covering the canopy (5) and the circumferential bordering element (6). The circumferential bordering element is present on all sides of the canopy (4) around the canopy top (5) but may consist of one or multiple elements that are joined together to form the circumferential bordering element (6). The canopy top (5) and the circumferential bordering element (6) define the partial volume (7) of the 3D space. Gravity is in opposite direction to the y-axis (-y) and the partial 3D space is open in this direction, i.e. not covered by any (structural) means, or covered with means that provide openings for air. Figure 1(b) shows a canopy (4) for use in the present invention in analogy to Figure 1(a) but in a round or elliptic form. The shape of the canopy (4) for use in the present invention is not limited and it can have any shape, regular or irregular, as long as it comprises a canopy top (5), a circumferential bordering element (6) and defines a partial volume (7). Also, the canopy (4) can be essentially air-tight or semi-permeable (see above explanations regarding membranes and fabrics) in opposite direction of gravity and essentially in z- and x-direction so that air does not escape the partial volume (7) in the aforementioned directions, at least not based on the natural (convective) movement of the air.

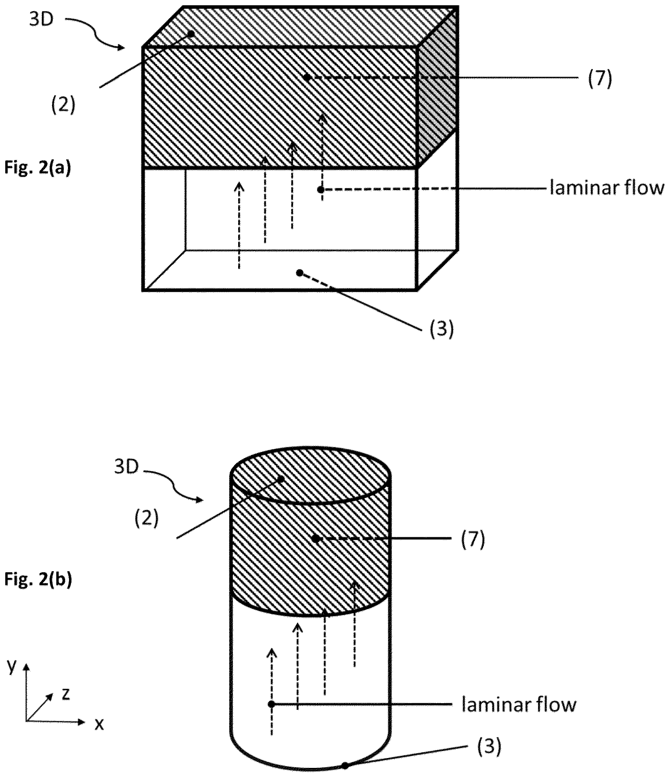

Figure 2(a) illustrates the 3D space and the partial volume (7) of the 3D space. The 3D space is the entire volume of the cube while the partial volume is the hatched area. The top (2) and bottom (3) of the 3D space are also indicated, wherein the opposite direction of the y-axis (-y) corresponds to gravity. The arrows inside the 3D space indicate the substantially laminar flow of air rising from the 3D space (optionally from the bottom (3) or an air outlet (not shown)) to the partial volume (7). Essentially, the top (2) of the 3D space corresponds to the canopy top (5) and the partial volume (7) corresponds to the volume defined by the canopy (4). The 3D space may have any shape and form, e.g. it can be of a cylindric form as shown in Figure 2(b), for which the above explanations apply mutatis mutandis.

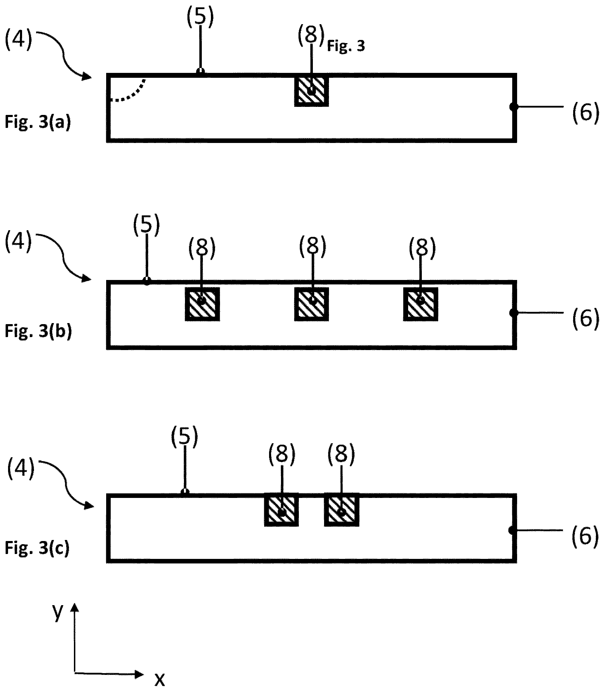

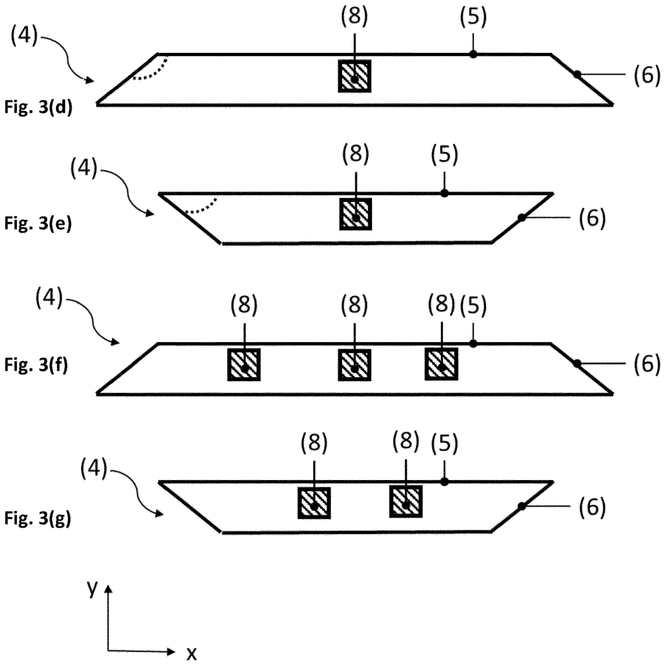

Figures 3(a) to (g) show different exemplary arrangements of the at least one air inlet member (8) in the partial volume formed by the canopy (4). All images are cross sections in in the y-,x-plane. One or more air inlet member(s) may be present and it/they may be positioned at any place within the canopy (4), e.g. attached to the canopy top (Figures 3(a), (c)) or offset from the canopy top (Figures 3(b), (d)-(g)) as long as the air inlet members are at least partially within the canopy (4) and/or are arranged such that they can take in air from the partial volume of the 3D space. The circumferential bordering element (6) can be positioned at any angle (shown as dotted line) relative to the canopy top (5), e.g. at a right angle (Figures 3(a)-(c)) or at an angle larger or smaller than 90 degrees (Figures 3(d)-(g)).

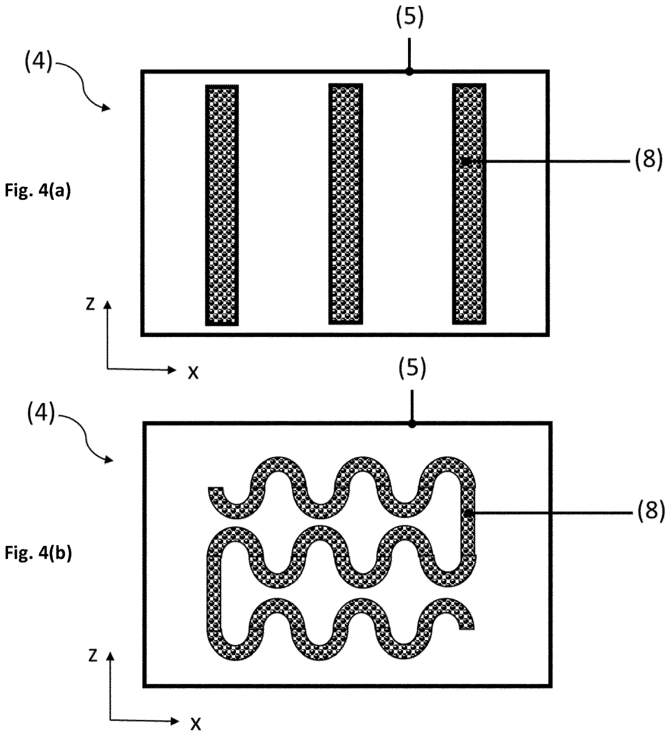

Figures 4(a) to (d) show different exemplary arrangements of the at least one air inlet member (8) in the canopy (4) with the canopy top (5) shown in the z-,x-plane in y direction (opposite to gravity). The air inlet members (8) can, e.g., be tubular and comprise multiple openings. The air inlet members (8) may be interconnected or individually connected to further parts of the system, e.g. the vertical support(s) (not shown). Any arrangement of the air inlet members is encompassed in the present invention as long as the arrangement allows for the substantially laminar flow of air to occur and for the air inlet members to take in air.

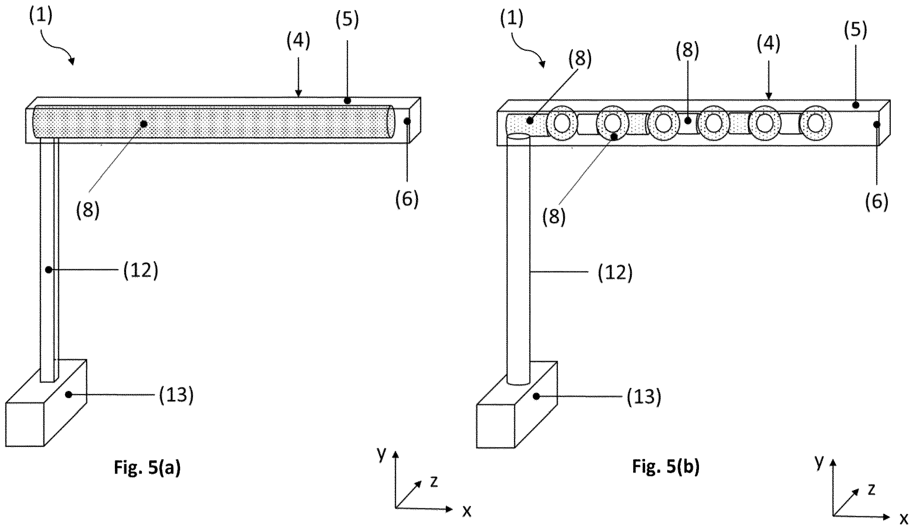

Figure 5(a) and (b) show different exemplary embodiments of the present system (1). The relation between canopy (4) dimensions and housing (13) dimensions are not limiting and the canopy (4) can cover a substantially larger area than depicted. A vertical support (12) connects the housing (13) with the at least one air inlet member (8) and optionally with the canopy (4). The vertical support can have any type of cross-section, e.g., round, elliptic or rectangular. The circumferential bordering element (6) may be longer in -y direction than indicated. As shown in Figure 5(b), the at least one air inlet member (8) can be formed by a network of tubes with sections that comprise holes and optionally sections without holes. The holes may also be distributed unevenly, with sections having a higher density of holes compared to sections with a relatively lower density of holes.

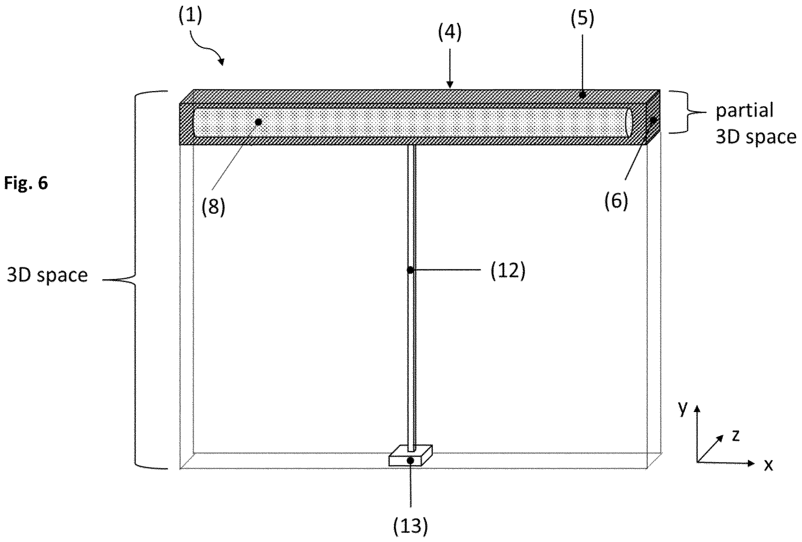

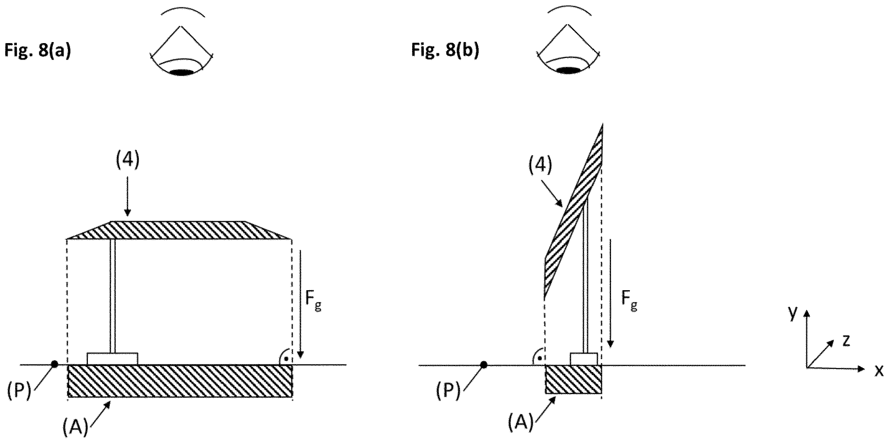

Figure 6 shows the relationship between the components of the system (1) and the 3D space and partial volume (7) of the 3D space. The 3D space is defined by (I) the height of the system (1), i.e. from the bottom/lowest point (e.g. the floor on which the cover (13) is positioned) of the system (1) to the canopy top (5), and by (II) the area formed by the canopy (4). The area of the canopy (4) corresponds to the area of the canopy top (5) if the system is configured as shown in this Figure (the circumferential bordering element is angled at 90 degrees relative to the canopy top and the canopy top is at 90 degrees relative to gravity). However, other angles are encompassed by the present invention and if the angle of the circumferential element is smaller or greater than 90 degrees, and/or if the canopy top is angled other than 90 degrees relative to gravity, the area of the 3D space equals the projection of the canopy (including any circumferential bordering elements angled at more or less than 90 degrees relative to the canopy top) in direction of gravity, i.e. in -y direction, on a plane that is perpendicular to gravity (z-,x-plane). Examples for this projection are shown in Figure 8.

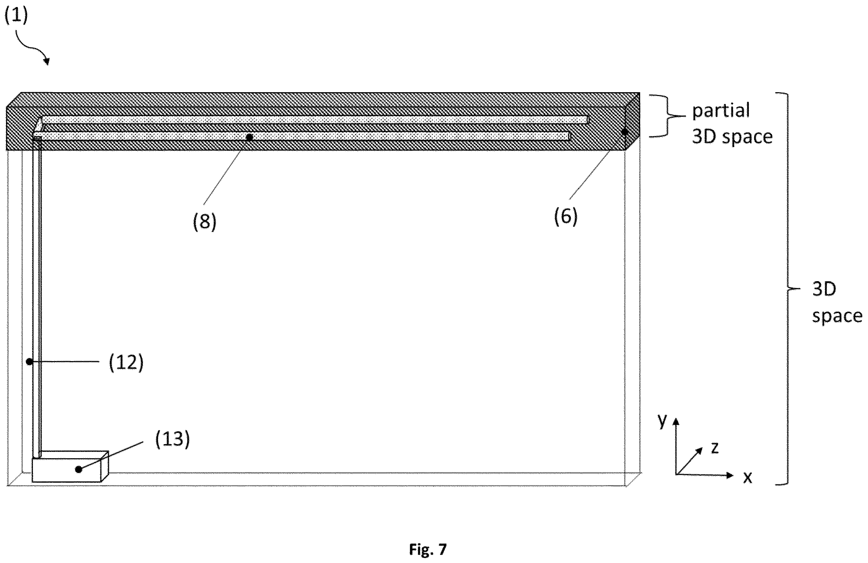

Figure 7 shows the relationship between the components of the system (1), the 3D space and partial volume (7) of the 3D space in analogy to Figure 6.

Figures 8(a) and (b) show the area of the 3D space as formed by the canopy (4). The plane that is perpendicular to gravity (z-, x-plane) is (P) and the area formed by the projection is (A). The area of the 3D space is dependent on the projection along gravity (illustrated by the eye on top of the Figures looking in -y direction) due to the nature of the substantially laminar flow from a bottom of the 3D space into the partial volume in a direction perpendicular to gravity.

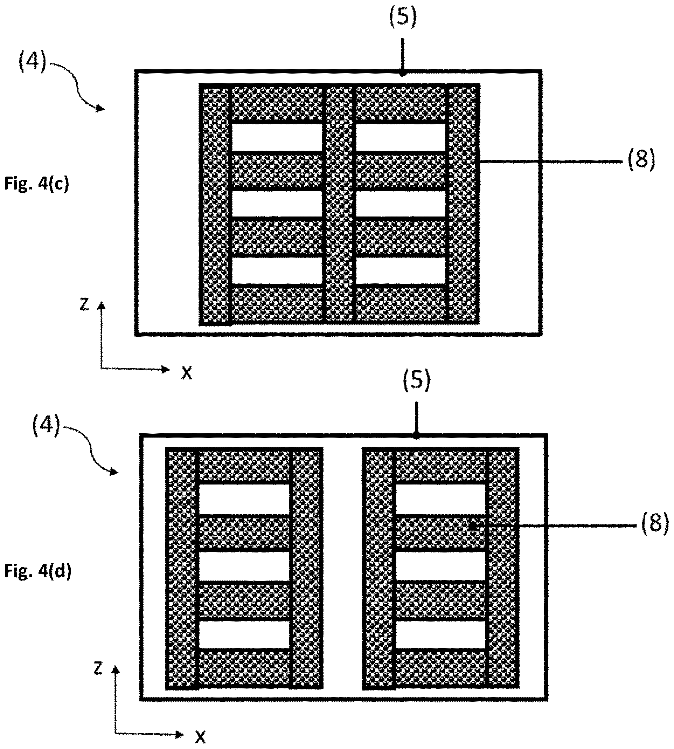

Figure 9 shows an exemplary embodiment of the present system (1) with a larger canopy essentially in analogy to Figure 5. For example, the air outlet (not shown) may be located in the housing (13) and configured to blow in x and -x direction. Of course, multiple and differently shaped air inlet member(s) may be present, e.g. as illustrated in Figures 3 and 4. For all exemplary embodiments shown in the Figures of the present invention, the shape or cross section of the housing (13), the vertical support (12) and the air inlet member (8) is not limiting, and any shape and cross section is encompassed as long as the function of the system (1) is achieved.

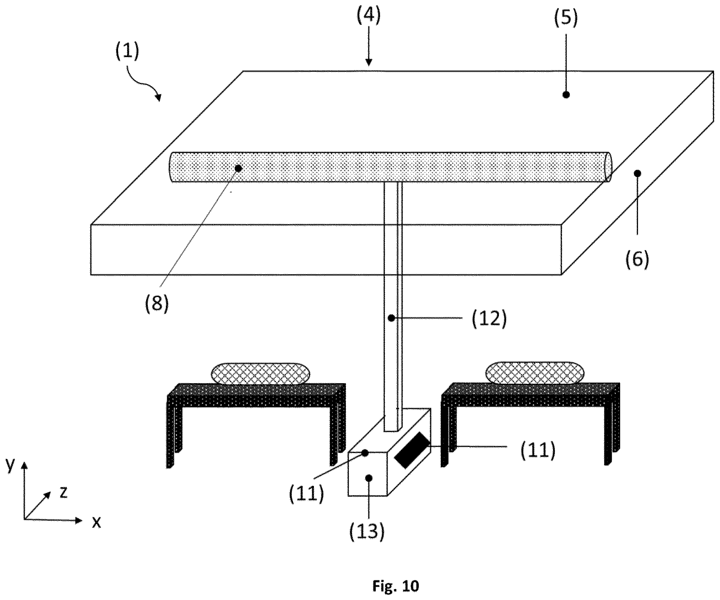

Figure 10 shows an exemplary embodiment of the present system (1) with a larger canopy essentially in analogy to Figure 9 with the air outlets (11) located in the housing (13) and configured to blow in x and -x direction. Of course, multiple and differently shaped air inlet member(s) may be present, e.g. as illustrated in Figures 3 and 4. Two beds are shown as exemplary locations for people in the 3D space. The substantially laminar flow (not shown) will emerge from the bottom (where the beds stand) and is at least partially fed by the air outlets, to rise into the canopy (4) where it is drawn in by the air inlet member (8) and is filtered and exhausted at least partially via air outlets (11). This circulation applies to all Figures shown herein. Another air outlet and another air inlet may be located outside the system (1), i.e. outside the 3D space in order to control, e.g., moisture, CO₂ and O₂ levels.

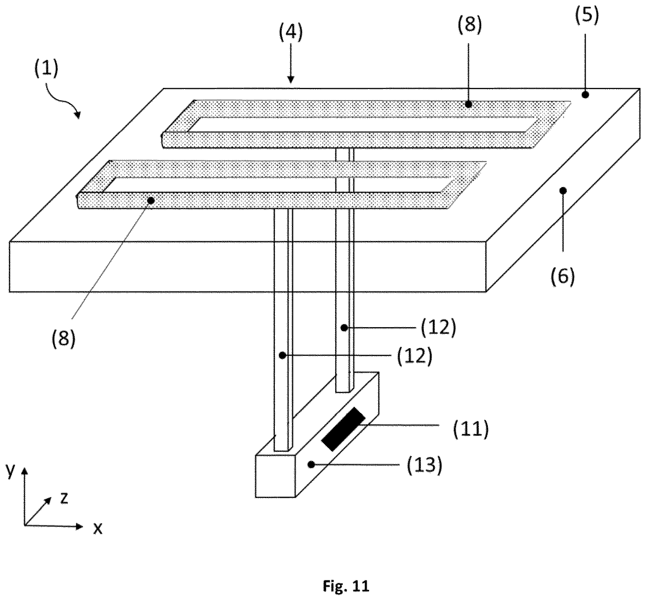

Figure 11 shows an exemplary embodiment of the present system (1) essentially in analogy to Figure 9 with two vertical supports (12) each connected to a circular air inlet member (8).

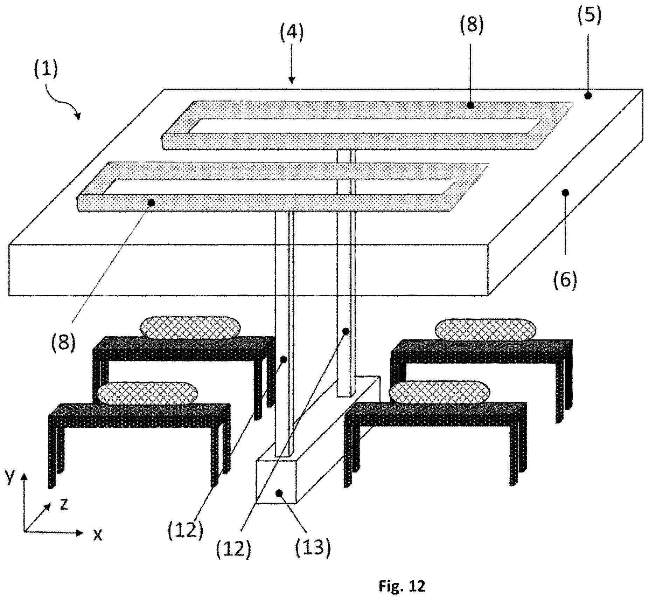

Figure 12 shows an exemplary embodiment of the present system (1) essentially in analogy to Figure 11 with four (less or more possible dependent on scale) beds within the 3D space. Air outlets (11) are not shown.

Figure 13 shows an exemplary embodiment of the present system (1) essentially in analogy to Figure 12 with the canopy (4) being positioned in L-shape relative to the vertical supports (12). This arrangement has the advantage that it can be positioned against a wall with the canopy (4) extending into the room away from the wall, as it would be advantageous in hospital or care home settings when a bed is positioned inside the 3D space (not shown). For example, the air inlet members (8) are attached to the vertical supports (12) and are essentially themselves capable of supporting the canopy (4) structure (including the air inlet members (8)). Additional diagonal support struts between the vertical support (12) and the air inlet member (8) can be installed but should not significantly block the accessibility of the 3D space from z, -z-, or -x-direction. In general, this assembly, e.g. together with a transparent canopy (4), allows for health personnel to reach a patient within the 3D space without obstacles, retains the room's own lighting and it also allows to keep the support infrastructure behind and on the side of the vertical supports (12). Also, the symmetrical arrangement of the vertical supports (12) and air inlet members (8) (together with, e.g., two filter devices and two air displacement devices, not shown) is highly effective in creating a laminar air flow around a person positioned in the middle of the symmetrical arrangement and warrants for redundancy because one half of the system (i.e. one air inlet member, one filter device and one air displacement device) would remain operational if one broke. Additionally, and for all embodiments described herein, a person entering, e.g. temporarily, e.g. care staff, can also profit from the air decontamination.

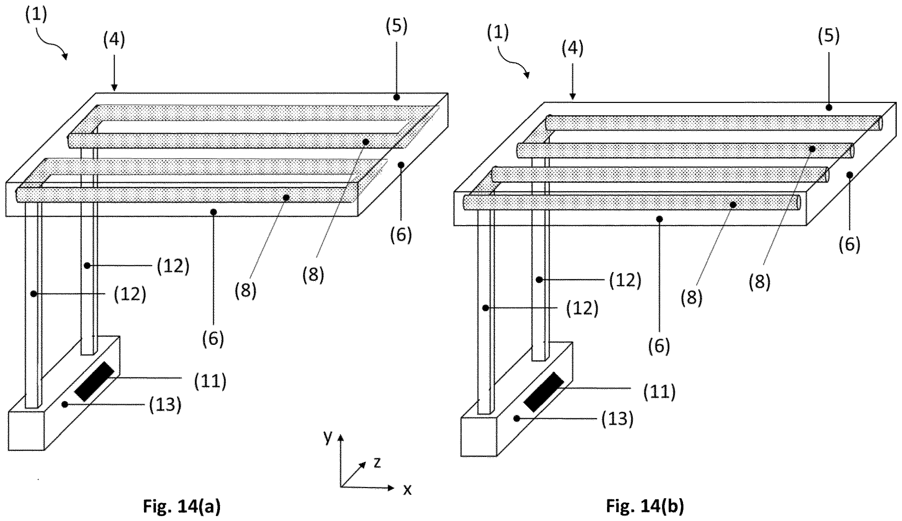

Figures 14(a) and (b) show exemplary embodiments of the present system (1) essentially in analogy to Figure 13 with different arrangements of the air inlet members (8). The air inlet members (8) may also be attached to the vertical supports (12) at a corner of the air inlet members (8). The arrangement of air inlet members (8) shown in these Figures can provide for an increased air intake along the central axis (x-direction) of the system (1) due to the presence of two air inlet member tubes (8), e.g. close to each other along this axis. An increased air intake along the central axis can be advantageous, e.g., if an infected person emitting ultrafine particles (e.g. viruses) is positioned in the middle of the 3D space, e.g. lying on a bed along the central axis, and the emitted particles emerging along the central axis can be efficiently taken up by the two air inlet members (8) along the central axis for decontamination.

Figure 15 shows an exemplary embodiment of the present system (1) essentially in analogy to Figures 14(a) and (b) with a higher level of detail. The circumferential bordering element (6) consists of multiple sections. The housing (13) comprises two air displacement devices (9) (e.g. fans) fluidly connected to each vertical support (12). The air displacement devices (9) are each fluidly connected in air flow direction to a filter device (10) (in the darker colored part of the housing (13)). The air outlet (11) is shown as a meshed structure exhausting air in x-direction. An optional further support element (16) is also shown in x-direction to add additional stability to the device. The diagonal and horizontal support struts attached to the at least one air inlet members (8) are optional and positioned such that they do not hinder access to the 3D space. As noted in the context of Figure 13, the air inlet members (8) are essentially themselves capable of supporting the canopy structure (including the air inlet members).

Figure 16 shows an exemplary embodiment of the present system (1) essentially in analogy to Figure 15 and further shows how a bed can be installed within the 3D space defined by the present system (1). In this exemplary embodiment, the air outlet (11) is positioned under the bed and in the center of the bed along the x-axis. This arrangement can be applied to all embodiments described herein and has the advantage that the air exiting the air outlet (11) supports the laminar flow around the bed (or other object with a contamination source) in y-direction and also in x-direction.

Figures 17(a) to (d) show exemplary embodiments of the air inlet member (8) being tubular and having openings distributed over around the tubes. In Figure 17(a), the openings are distributed over about 120 degrees (angle α) around the tubular air inlet member (8). If the openings are distributed over about 120 to 179 degrees (120 degrees shown in Figure 17(a)), it is preferred that the openings are positioned such that they face the 3D space and not the canopy top, as illustrated by the openings facing the direction of gravity. In this optional embodiment, the openings can be rotated to either side, optionally as long as the majority of the openings faces the direction of gravity. If the openings are distributed over about 180 degrees (Figures 17(b) and (c)) or over about 240 degrees (Figure 17(d)) they can be oriented such that air can be drawn in from a direction facing the canopy top (y-direction), from an essentially horizontal direction (x-direction), as well as from a direction facing the 3D space (direction of gravity, -y).

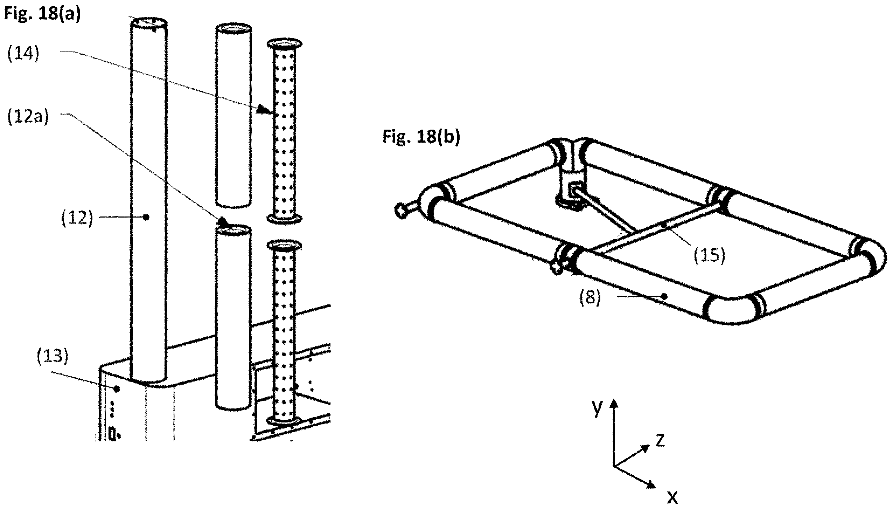

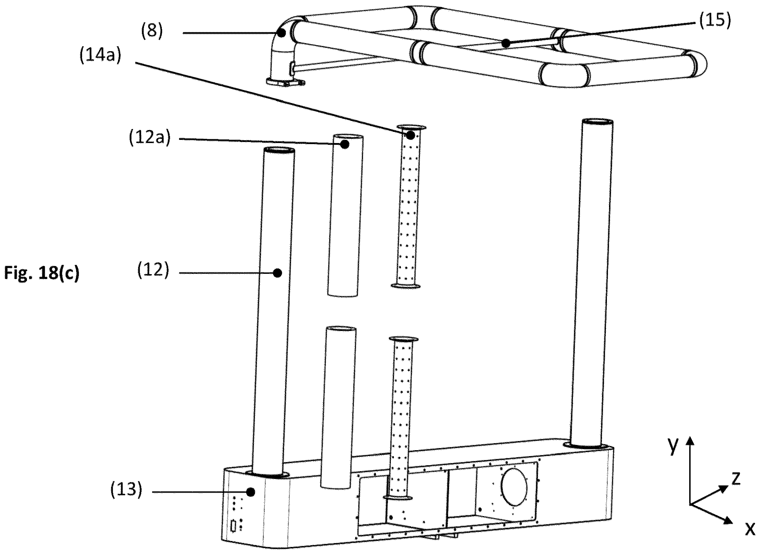

Figure 18(a) shows a close-up of the vertical support (12) and its optional contents. The vertical support (12) may be made of individual parts (2 shown, more are encompassed) (12a) which may be detachable and/or it or the individual parts may be telescopic. Independent of these properties, and for all embodiments of the vertical support for use in the present system, the vertical support (12) may comprise means for noise reduction (14) inside the vertical support. The means for noise reduction (14) can be inserted into the vertical support (12) and may act as resonance or absorption elements, wherein the absorption layers are adapted in their geometrical depth (optionally about 30 to 100 mm) and/or density to the frequency response in order to, e.g., reduce harmonic small band noise (e.g. harmonic or fan blade frequency) and optionally create a sound spectrum that is more pleasant for the human ear. Figure 18(b) shows an exemplary air inlet member (8) where the openings for air intake in the air inlet member are not shown. The air inlet member may comprise optional support struts (15) to further increase stability of the air inlet member (8). The flange on the top corner of the air inlet member (8) is configured to be fluidly connected with a vertical support (not shown). Also shown are optional fittings for structurally connecting two of the air inlet members shown (8) in Figure 18(b) (tubes with a cross). Figure 18(c) shows an exemplary combination of the elements described for Figures 18(a) and 18(b), wherein the corner of the air inlet member (8) is connected to the vertical support (12). A second air inlet member (mirrored along the x-axis, not shown) can be connected to the second vertical support shown in Figure 18(c).

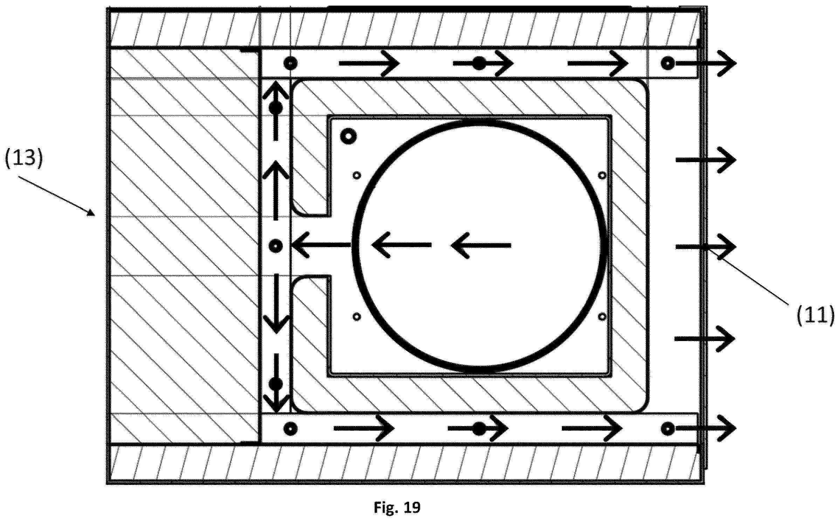

Figure 19 shows an exemplary embodiment of means for noise reduction (14), hashed area, for example in the housing (13). The means, the housing, the air displacement device (round circle) and/or filter device (round circle) can be configured to deflect the air exiting the filter device (10) and/or the air displacement device (9) by about 180 degrees through the means for noise reduction (14) to the air outlet (11). The air flow is indicated by arrows.

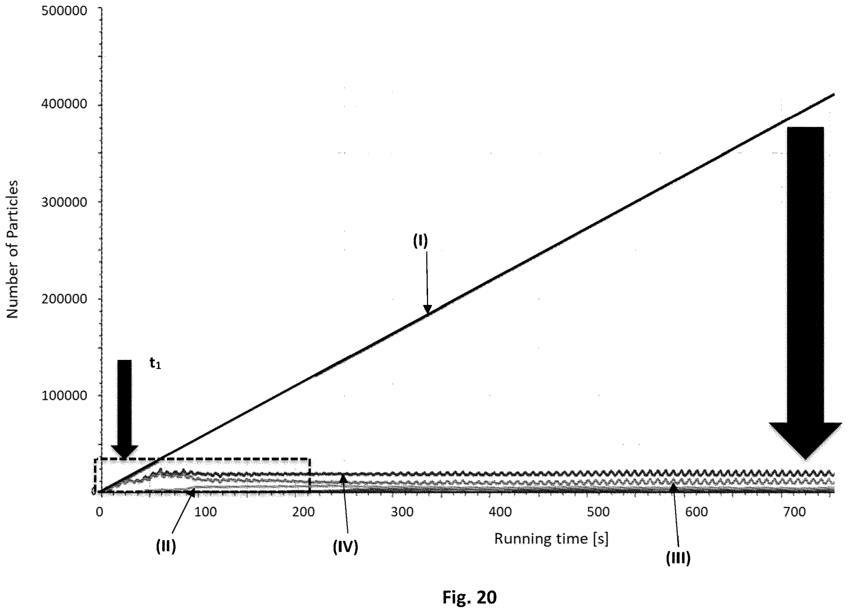

Figure 20 shows the number of viruses in the 3D space of a system according to the present invention relative to time, as modelled in a computer simulation. The system was modelled to be operated at 140 m³/h and was based on a configuration shown in Figure 15. It was assumed that 5000 virus particles are emitted per breath of a person in the system. (I) refers to the total number of viruses emitted over time by a person in the 3D space without filtration/activation of the system according to the present invention. (II)-(IV) show virus numbers after decontamination of the 3D space was started (arrow t₁). (II) refers to the number of viruses on the sides of the 3D space. (III) refers to the number of viruses in a central area of the 3D space and (IV) refers to the total number of viruses in the 3D space. The big arrow at about 700 s shows the reduction of the virus count in the 3D space by the system of the present invention.

## Claims

1. A system (1) for air decontamination of a three-dimensional (3D) space having a top (2) and a bottom (3) being offset in direction of gravity with respect to the top (2), the system (1) comprising
(I) at least one canopy (4) comprising a canopy top (5) positioned essentially at the top (2) of the 3D space and bordered by a circumferential bordering element (6) extending from the canopy top (5) towards the 3D space, wherein the canopy top (5) and the bordering element (6) define a partial volume (7) of the 3D space,
(II) at least one air inlet member (8) positioned in the partial volume (7), and configured to draw in air from the partial volume of the 3D space and optionally from the 3D space, and
(III) an air displacement device (9) configured to move air from the at least one air inlet member (8) through a filter device (10) and through at least one air outlet (11) into the 3D space, optionally into the 3D space essentially at the bottom (3) of the 3D space, wherein the filter device (10) is configured to filter ultrafine particles from the air drawn in through the at least one air inlet member (8) and optionally filter ultrafine particles from fresh air,
wherein the system (1) is configured to draw air in a substantially laminar flow essentially in direction of the bottom (3) of the 3D space to the top (2), optionally essentially from the bottom (3) to at least the partial volume (7).

2. The system (1) according to claim 1, wherein the at least one air inlet member (8) is positioned essentially at the canopy top (5) and is optionally at least partially attached to the canopy top (5).

3. The system (1) according to claim 1 or 2, wherein the at least one air inlet member (8) is formed by tubes, optionally positioned essentially in parallel to the canopy top (5), optionally by tubes comprising openings distributed over about 120 to 360 degrees around the tubes, for drawing in air.

4. The system (1) according to any of claims 1 to 3, wherein the system (1) is configured to draw in about 100 to 200 m³ of air per hour.

5. The system (1) according to any of claims 1 to 4, wherein
- the system is configured so that the substantially vertical laminar flow has an average speed of about 0.002 to 0.04 m/s, optionally 0.005 to 0.02 m/s,
- the total area of all openings in the air inlet member (8) for air intake, optionally the sum of all opening cross-sections in the air inlet member (8), is about 0.009 to 0.036 m², optionally about 0.0015 to 0.025 m², for a volumetric flow within the system of about 45 to 180 m³/h, optionally for about 80 to 120 m³/h, and/or
- the total area of all openings in the air outlet (11) for air outlet, optionally the sum of all opening cross-sections in the air outlet (11), is about 0.0135 to 0.054 m², optionally about 0.015 to 0.03 m², for a volumetric flow within the system of about 45 to 180 m³/h, optionally for about 80 to 120 m³/h.

6. The system (1) according to any of claims 1 to 5, wherein the at least one air inlet member (8) is formed by tubes comprising openings, optionally distributed over about 120 to 360 degrees around at least parts of the tubes, wherein
- the porosity of the tubes formed by the openings is about 0.5 to 2 %,
- the openings have an area of about 2 to 4 mm², optionally a diameter of about 1 to 4 mm or about 1.5 to 2.5 mm for round openings,
- the total volume of the at least one air inlet member (8) formed by tubes is about 2.5 to 5 % of the volume of the 3D space, and/or
- the cross-sectional area of the at least one air inlet member tube is about 0.5 to 2 times larger than the total area of the openings.

7. The system (1) according to any of claims 1 to 6, wherein the at least one air inlet member (8) forms a support structure for the at least one canopy (4) and optionally wherein the canopy top (5) and/or the circumferential bordering element (6) is/are transparent.

8. The system (1) according to any of claims 1 to 7, wherein the canopy top (5) is essentially flat, and/or optionally wherein the circumferential bordering element (6) extends at an angle of about 45 to 135 degrees, optionally about 90 degrees, relative to the canopy top (5).

9. The system (1) according to any of claims 1 to 8, wherein the at least one canopy (4), optionally the at least one air inlet member (8) attached to the canopy top (5) and/or the circumferential bordering element (6), is connected to at least one essentially vertical support (12) for positioning and supporting the at least one canopy (4) above the bottom (3) of the 3D space.

10. The system (1) according to any of claims 1 to 9, wherein the vertical support tube (12) supports and fluidly connects the at least one air inlet member (8) with a housing (13) comprising the filter device (10) and the air outlet (11) and optionally the air displacement device (9), wherein the housing (13) is optionally positioned on the bottom (3) of the 3D space.

11. The system (1) according to any of claims 1 to 10, wherein the vertical support (12) and/or the housing (13) comprise(s) means for noise reduction, optionally wherein the means for noise reduction in the housing (13) are configured to deflect the air exiting the filter device (10) and/or the air displacement device (9) by about 180 degrees through the means for noise reduction to the air outlet (11).

12. The system (1) according to any of claims 1 to 11, wherein the system is configured to accommodate a bed in the 3D space, optionally wherein the housing (13) is configured to be positioned behind and below the head section of the bed.

13. The system (1) according to any of claims 1 to 12, wherein the system comprises
- two air inlet members (8) each formed by a tube loop and comprising openings distributed over about 120 to 360 degrees around at least part of the tube,
- the canopy (4) supported by the two air inlet members (8),
- two vertical supports (12) each fluidly connected to and supporting one of the two air inlet members (8) and both further fluidly connected to
- the housing (13) comprising the filter device (10) and the air outlet (11), and optionally the air displacement device (9) .

14. The system (1) according to claim 13, wherein
- the two vertical supports (12) each define an adjacent corner of the 3D space and together with the housing (13) and the canopy (4) essentially define the height of the 3D space; and/or
- the canopy top (5), the circumferential bordering element (6), and/or the at least one air inlet member (8) is/are detachably, pivotably or foldably connected to each other and/or to the at least one essentially vertical support (12) .

15. A kit of parts comprising the components of the system (1) according to any of claims 1 to 14, and optionally instructions for their assembly.
